# EUROPÄISCHE PATENTSCHRIFT

(11) **EP 1 487 361 B1**
(45) Veröffentlichungstag und Bekanntmachung des Hinweises auf die Patenterteilung: **08.08.2007**
(21) Anmeldenummer: 03714892.1
(22) Anmeldetag: 27.03.2003
(51) Int. Cl.: A61B 17/68, A61B 17/58

(54) **EINRICHTUNG ZUR STABILISIERUNG EINES BRUCHES EINES RÖHRENKNOCHENS**
DEVICE FOR STABILISING A BREAK IN A TUBULAR BONE
DISPOSITIF POUR STABILISER UNE FRACTURE DANS UN OS LONG

(30) Priorität: 27.03.2002 AT 4812002
(43) Veröffentlichungstag der Anmeldung: 22.12.2004
(73) Patentinhaber: Seyr, Volkmar, 6060 Ampass (AT); Goreis, Helmut, 6020 Innsbruck (AT)
(72) Erfinder: Seyr, Volkmar, 6060 Ampass (AT); Goreis, Helmut, 6020 Innsbruck (AT)
(74) Vertreter: Haffner, Thomas M.
(86) Internationale Anmeldenummer: PCT/EP2003/003213
(87) Internationale Veröffentlichungsnummer: WO 2003/079913

(56) Entgegenhaltungen:
- EP-A- 0 401 650
- FR-A- 2 726 172
- FR-A- 2 818 888
- US-A- 5 300 075

## Beschreibung

Die vorliegende Erfindung betrifft eine Einrichtung mit mindestens einem biegsamen Führungsdraht oder dergleichen zur Stabilisierung eines Bruches eines Röhrenknochens, wobei der Führungsdraht innerhalb der Röhre des Röhrenknochens verschiebbar ist.

Beim Stand der Technik sind verschiedenste Möglichkeiten zur Stabilisierung von Brüchen von Röhrenknochen wie z.B. dem Oberarmknochen oder dem Oberschenkelknochen bekannt. Die verschiedenen Vorgehensweisen verwenden Metallplatten, Metallstifte, Marknägel sowie gattungsgemäße Spiraldrähte. Die beim Stand der Technik bekannten Behandlungsmethoden weisen jedoch verschiedenartige Probleme und Nachteile auf. So ist bei der Verwendung von Metallplatten der Operationsaufwand sehr groß und der medizinische Eingriff relativ aufwendig. Bei der Verwendung von Marknägeln ist anzuführen, dass nicht alle Bruchformen hiermit stabilisiert werden können und darüber hinaus der Operationsaufwand sehr groß ist. Beim Stand der Technik ebenfalls bekannte einfache Bohrdrähte rutschen häufig schon nach relativ kurzer Zeit selbstständig aus dem Knochen heraus und führen dadurch zu Misserfolgen in der Behandlung des Knochenbruches. Bei der Verwendung des gattungsgemäßen Spiraldrahtes liegt zunächst eine relativ schnell durchführbare Operationsmethode vor. Die Erfahrung zeigt jedoch, dass bei der Verwendung von Spiraldrähten die gebrochenen Teile des Knochens beim Verheilen nicht ausreichend aufeinander zurutschen können, wodurch das Zusammenheilen des Knochens negativ beeinträchtigt wird, was letztendlich zu einer erhöhten Pseudoarthrose führt.

Die Merkmale des Oberbegriffs des Anspruchs 1 sind aus dem Dokument EP-A-0 401 650 bekannt.

Aufgabe der vorliegenden Erfindung ist es somit, eine Einrichtung zu schaffen, die neben einer Verkürzung der Operationszeit und Verminderung des Operationsaufwandes zu einer Beseitigung der oben angeführte Nachteile führt.

Dies wird erfindungsgemäß durch die Merkmale des Anspruchs 1 erreicht.

Durch das erfindungsgemäße Vorhandensein des abgerundeten Kopfes kann der biegsame Führungsdraht durch eine kleine Öffnung in der Haut bzw. im Muskel und eine vorzugsweise schräge Bohrung im Röhrenknochen in die Röhre des Knochens eingebracht und anschließend in dieser zur Bruchstelle des Knochens geschoben werden. Hierbei bedingt der abgerundete Kopf, dass die Einrichtung besonders gut an der Innenwandung des Röhrenknochens entlang rutscht. Dies ermöglicht ein besonders einfaches und schnelles Einführen der Einrichtung in den Knochen. Hat der Kopf seine endgültig vorhergesehene Position erreicht, so ist der gebrochene Röhrenknochen ausreichend stabilisiert und kann in dieser Position verheilen. Wenn der Bruch wieder zusammengewachsen ist, kann die Einrichtung durch Herausziehen wieder entfernt werden. Nach dem Entfernen der erfindungsgemäßen Einrichtung wachsen die nur kleinen Öffnungen in der Haut, im Muskel und im Knochen durch die die Einrichtung eingeführt und wieder gezogen worden ist, schnell zusammen. Insgesamt ermöglicht die erfindungsgemäße Einrichtung eine schnelle und sichere Operationsmethode zur Behandlung von Knochenbrüchen bei Röhrenknochen. Dies ist besonders bei älteren Menschen und kleineren Kindern günstig, da durch die kurzen Operationszeiten die mit dem Eingriff verbundenen Komplikationen auf einem Minimum gehalten werden können. Durch die Kürze des Eingriffs wird darüber hinaus noch die Röntgendurchleuchtungszeit verkürzt, was wiederum zu einer reduzierten Strahlenbelastung des zu behandelnden Patienten und des Operierenden führt.

Zum einfachen Einführen und Herausziehen der erfindungsgemäßen Einrichtung ist es günstigerweise vorgesehen, dass die Einrichtung am abgerundeten Kopf den größten Durchmesser aufweist. Zur Behandlung von Oberarmknochenbrüchen oder Oberschenkelknochenbrüchen bei Menschen sehen hierbei ausgewählte Ausführungsvarianten vor, dass der Kopf einen größten Durchmesser zwischen 0,4 cm und 1,6 cm, vorzugsweise zwischen 0,4 cm und 1,0 cm aufweist. Hierbei ist jedoch darauf hinzuweisen, dass der Durchmesser des Kopfes an den Durchmesser der Röhre des jeweils zu behandelnden Knochens angepasst sein muss. Zur Stabilisierung anderer menschlicher oder tierischer Röhrenknochen können daher auch andere Kopfdurchmesserbereiche vorgesehen sein.

Zur weiteren Verbesserung der Fixierung bzw. Stabilisierung des Knochenbruches ist in einer günstigen Ausführungsvariante vorgesehen, dass die Einrichtung zusätzlich mindestens einen Verankerungsdraht, vorzugsweise drei oder mehrere Verankerungsdrähte, oder dergleichen aufweist. Der Verankerungsdraht bzw. die Verankerungsdrähte werden nach dem Einbringen der Einrichtung in den Röhrenknochen weiter nach vorne über den Kopf hinaus geschoben und in den Knochen geschlagen bzw. in diesem verspreizt. Hierdurch ist auch bei schlechter Knochenqualität ein Herausrutschen der Einrichtung wirkungsvoll verhindert.

Zum besonders einfachen Einführen der Einrichtung in den Knochen ist es vorgesehen, dass der Verankerungsdraht, vorzugsweise alle Verankerungsdrähte, zwischen mindestens einer Position bei der sein (ihr) kopfseitiges Ende hinter dem größten Durchmesser des Kopfes verborgen ist und mindestens einer Position bei der sein (ihr) kopfseitiges Ende über den Kopf hinausragt, verschiebbar ist (sind). So sind die Verankerungsdrähte in dieser Ausführungsvariante beim Einführen der Einrichtung in den gebrochenen Röhrenknochen zunächst hinter dem Kopf verborgen. Erst nachdem der Führungsdraht bzw. dessen abgerundeter Kopf seine endgültige Position erreicht hat, werden die Verankerungsdrähte über den Kopf hinweg in den Knochen geschoben bzw. geschlagen und dabei aufgespreizt. Dies führt wiederum zu einer besonders haltbaren und sicheren Stabilisierung des Knochens. Hierbei kann der Kopf des Führungsdrahtes im Röhrenknochen sowohl über die Bruchstelle hinweg geschoben werden, als auch kurz vor dieser angeordnet sein. Ist die Einrichtung über den Bruch hinweg eingeschoben, so dienen die Verankerungsdrähte der zusätzlichen Stabilisierung. Liegt der Bruch jenseits der Endposition des Kopfes des Führungsdrahtes, kann eine ausreichende Stabilisierung des Bruchs auch durch die Verankerungsdrähte alleine erreicht werden.

Weitere Merkmale und Einzelheiten ergeben sich aus der nachfolgenden Figurenbeschreibung. Dabei zeigt:
- Fig. 1: ein erfindungsgemäßes Ausführungsbeispiel mit hinter den Kopf zurückgezogenen Verankerungsdrähten,
- Fig.2: das in Fig. 1 gezeigte Ausführungsbeispiel mit über den Kopf hinweg geschobenen Verankerungsdrähten,
- Fig. 3a, 3b, 4 und 5: verschiedene Detailansichten einzelner Bauteile des in den Fig. 1 und 2 gezeigten Ausführungsbeispiels,
- Fig. 6 und 7: Beispiele für die Anordnung der erfindungsgemäßen Ausführungsform in gebrochenen Röhrenknochen,
- Fig. 8: das in Fig. 1 gezeigte Ausführungsbeispiel mit einem in den Kopf geschnittenen Gewinde, und
- Fig. 9: eine Seitenansicht einer weiteren Ausführungsform der erfindungsgemäßen Einrichtung mit teilweise geschnittenen und teilweise weggebrochenen Teilen.

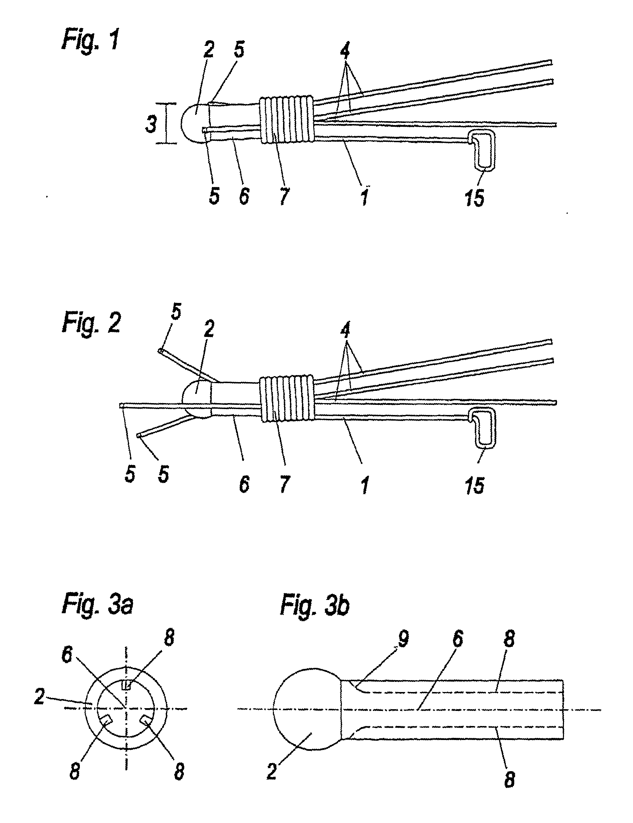

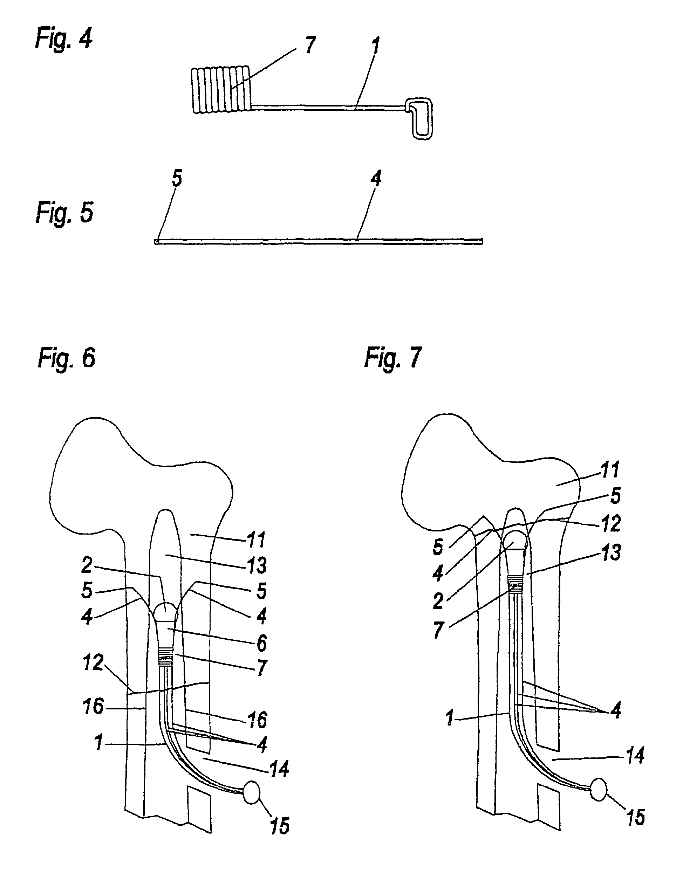

Das in den Fig. 1 und 2 gezeigte Ausführungsbeispiel besitzt einen abgerundeten Kopf 2 sowie einen Führungsdraht 1 dessen kopfseitiges Ende als Spiralfeder 7 ausgebildet ist (siehe hierzu auch Fig. 4). Am entgegengesetzen Ende des Führungsdrahtes 3 ist eine Griffschlaufe 15 zur besseren Handhabung vorgesehen. Der Kopf 2 weist an seinem zum Führungsdraht 1 weisenden Ende einen vorzugsweise im wesentlichen zylinderförmigen bzw. kolbenförmigen Grundkörper 6 auf. Der Grundkörper 6 ist an seinem zum Führungsdraht 1 weisenden Ende von der biegsamen Spiralfeder 7 zumindest teilweise umfasst. Zur leichteren Montage kann hierbei vorgesehen sein, dass der Grundkörper 6 mit einem Gewinde versehen ist, auf das die Spiralfeder 7 aufgedreht werden kann. Der Grundkörper 6 weist wie in den Fig. 3a und 3b im Detail dargestellt im Bereich hinter dem größten Durchmesser 3 des Kopfes 2 im gezeigten Ausführungsbeispiel 3 Nuten auf, die zur Führung jeweils mindestens eines Verankerungsdrahtes vorgesehen sind. Bei der Verwendung von mehr oder weniger als 3 Verankerungsdrähten ist eine entsprechende Anzahl von Nuten 8 vorzusehen. Die Nuten 8 sind, wie in den Fig. 3a und 3b in zwei verschiedenen Perspektiven gezeigt, in den im wesentlichen zylinderförmigen Grundkörper 6 in dessen Längsrichtung geschnitten und enden hinter dem Kopf 2 in einem jeweils bogenförmigen Auslauf 9. Dieser Auslauf 9 kann in seiner Form günstigerweise als Teil eines Kreissegmentes ausgeführt sein. Die Verankerungsdrähte 4 sind in den Nuten 8 geführt und werden im hinteren Bereich des Grundkörpers 6 von der Spiralfeder 7 umfasst, sodass sie hier zwischen der Spiralfeder 7 und dem Grundkörper 6 gehalten sind. Beim Einführen des gezeigten Ausführungsbeispiels in den Knochen sind die Verankerungsdrähte 4 zunächst, wie in Fig. 1 gezeigt, so weit zurückgezogen, dass ihre kopfseitigen Enden bzw. Spitzen 5 hinter dem größten Durchmesser 3 des Kopfes 2 in den Nuten 8 verborgen liegen. Dies ermöglicht ein besonders gutes Entlanggleiten des Kopfes 2 bzw. des der gesamten Einrichtung an der Innenwandung 16 der Röhre 13 des Röhrenknochens 11. Hat der Kopf 2 bzw. die gesamte Einrichtung die gewünschte Position im Röhrenknochen erreicht, so werden die Verankerungsdrähte 4 mit ihren kopfseitigen Enden 5 über den Kopf 2 hinaus in den die Einrichtung umgebenden Knochen 11 getrieben bzw. geschlagen. Fig. 2 zeigt diese Stellung der Verankerungsdrähte 4. Beim Hinausschlagen wird durch die bogenförmige Ausgestaltung der Abschlüsse 9 der Nuten 8 ein möglichst großer Spreizwinkel der Verankerungsdrähte 4 erreicht. Fig. 5 zeigt einen Verankerungsdraht 4 mit dem günstigerweise als Spitze ausgebildeten kopfseitigen Ende 5.

Die Fig. 6 und 7 zeigen in einer Schnittdarstellung eines Knochens 11 zwei Beispiele wie die anhand der Fig. 1 bis 5 erläuterte erfindungsgemäße Ausführungsform im Knochen zur Stabilisierung des an der Bruchstelle 12 gebrochenen Knochens angeordnet und verankert werden kann. Im Zuge des operativen Eingriffs zur Stabilisierung des Knochenbruches wird zunächst eine Öffnung in der Haut und dem Muskel sowie ein Bohrloch 14 im Knochen 11 zur Einführung der erfindungsgemäßen Einrichtung geschaffen. Nach Ausrichtung des Knochenbruchs erfolgt anschließend das Einschieben, Einschlagen bzw. Eindrehen der erfindungsgemäßen Einrichtung über das Bohrloch 14 in den Markraum bzw. die Röhre 13 des Röhrenknochens 11. Die Bohrung 14 ist hierbei günstigerweise schräg ausgebildet, damit der Kopf 2 beim Einführen in die Röhre 13 bereits in einem für das Entlanggleiten an der Innenwandung 16 besonders günstigen Winkel auf diese auftrifft. Die Verankerungsdrähte 4 sind während des Einschiebens hinter den Kopf 2 zurückgezogen, wie dies in Fig. 1 dargestellt ist. Durch den abgerundeten Kopf 2 wird erreicht, dass die erfindungsgemäße Einrichtung bei ihrem Vorschieben auf die gewünschte Endposition gut an der Innenwandung 16 der Röhre 13 entlanggeleitet. Ist die Endposition der erfindungsgemäßen Einrichtung erreicht, so erfolgt die Kontrolle der Bruchstellung und der Lage der erfindungsgemäßen Einrichtung im Knochen z.B. mittels Röntgenwandler. Anschließend werden die drei im gezeigten Ausführungsbeispiel vorhandenen in den Nuten 8 verlaufenden Verankerungsdrähte 4 über den Kopf 2 hinweg vorgeschlagen. Sie verspreizen sich dabei aufgrund der bogenförmigen Abschlüsse 9 der Nuten 8 pyramidenförmig im Knochen. Die Fixierung bzw. Stabilisierung des gebrochenen Röhrenknochens 11 erfolgt somit zunächst durch das Einführen der erfindungsgemäßen Einrichtung in die Röhre des Knochens. Eine weiter verbesserte Stabilisierung des Knochens erfolgt anschließend durch das Hinausschlagen der Verankerungsdrähte 4. Die Spiralfeder 7 mit ihrer relativ glatten Oberfläche erlaubt ein Zusammensinken der Bruchstücke des Knochens 11. Außerdem führt sie aufgrund ihrer Elastizität zu einem weiteren Verspreizen der erfindungsgemäßen Einrichtung.

Fig. 6 zeigt einen Röhrenknochen, bei dem die erfindungsgemäße Einrichtung in der Röhre 13 über die Bruchstelle hinweg geschoben wurde. Nachdem diese Position erreicht ist, erfolgt die Verankerung der Verankerungsdrähte 4 im Knochen 11. Fig. 7 zeigt ein Beispiel, bei dem der kopf 2 in seiner Endposition unterhalb der Bruchstelle 12 zu liegen kommt. Die Verankerung der Bruchstücke des Knochens 11 erfolgt bei dieser Anwendung ausschließlich über die pyramidenförmig aufgespreizt in den Knochen zu schlagenden Verankerungsdrähte 4.

Aus der in Fig. 6 und 7 dargestellten Situation ist ersichtlich, dass die erfindungsgemäße Einrichtung sowohl bei Knochenbrüchen im Bereich der Röhre sowie auch bei Knochenbrüchen außerhalb der Röhre 13 des Röhrenknochens 11 verwendet werden kann. Hierbei ist darauf hinzuweisen, dass die erfindungsgemäße Einrichtung den jeweils zu stabilisierenden Röhrenknochen entsprechend dimensioniert und diese Art der Behandlung von Knochenbrüchen sowohl beim Menschen wie auch bei Tieren angewendet werden kann.

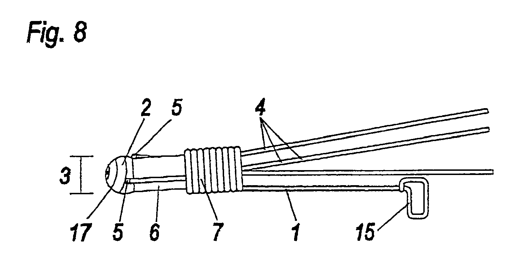

Der abgerundete Kopf weist in der in Fig. 1 gezeigten Variante eine glatte Oberfläche auf. Dies ermöglicht ein besonders gutes Abgleiten an der Innenwandung 15 des Röhrenknochens 11. Alternativ kann wie in Fig. 8 gezeigt, jedoch auch vorgesehen sein, dass der abgerundete Kopf 2 an seiner Oberfläche eine gewindeförmige Strukturierung oder ein Gewinde 17, vorzugsweise mit geringer Steigung, aufweist. Hierdurch wird erreicht, dass durch Drehen der Einrichtung das Gewinde oder die gewindeförmige Struktur zu einem gezielten Abgleiten des Kopfes 2 an der Innenwandung 15 beiträgt. Dies ist besonders dann günstig, wenn der Kopf 2 in einem sehr spitzen Winkel auf die Innenwandung 15 trifft. Das Gewinde oder die gewindeförmige Struktur ist hierbei in der Art ausgebildet, dass sie den Kopf ablenkt, aber nicht den Kopf einschneidet.

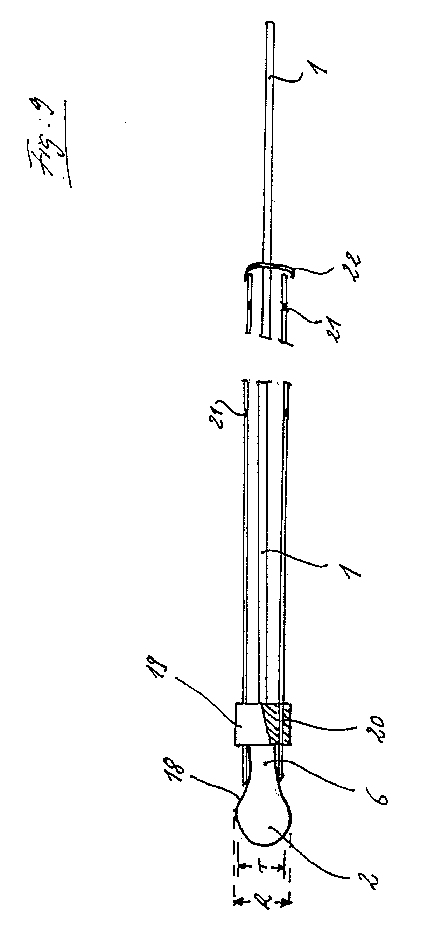

Bei der Ausführungsform nach Figur 9 sind der rotationssymmetrische Grundkörper 6 und der halbkugelförmige Kopf 2 einstückig ausgebildet. Der Grundkörper 6 weist dabei eine sich zum Kopf 2 hin kontinuierlich erweiternde Führungsfläche 18 auf. Der Führungsdraht 1 ist an dem vom Kopf 2 abgewandten Ende an dem Grundkörper 6 fixiert, beispielsweise durch eine Axialbohrung im Grundkörper 6, in die der Führungsdraht 1 ragt.

Zur Führung der drei oder vier mit gleichem Winkel versetzt angeordneten Verankerungsdrähte 4 ist an dem vom Kopf 2 abgewandten Ende des Grundkörpers 6 ein Ring 19 vorgesehen, der mit Längsbohrungen 20 versehen ist, durch die die Verankerungsdrähte 4 gesteckt werden. Der Ring 19 weist einen Außendurchmesser r auf der kleiner ist als der Außendurchmesser R des Kopfes 2.

Um die optische Trennung zu erhöhen, sind die Verankerungsdrähte 4 an dem vom Kopf 2 abgewandten Abschnitt mit Einkerbungen 21 versehen. An dem vom Kopf 2 abgewandten Ende der Verankerungsdrähte 4 in der in Fig. 9 dargestellten Einführposition ist auf dem Führungsdraht 1 eine schüsselförmig ausgebildete Scheibe 22 vorgesehen. An ihr werden die Verankerungsdrähte 4 in der Einführposition gehalten. Der sich an der Scheibe 22 anschließende Endabschnitt 23 des Führungsdrahtes 1 dient zugleich als Handgriff.

Der Führungsdraht 1 und die Verankerungsdrähte 4 bestehen vorzugsweise aus Metall, beispielsweise einer Titanlegierung oder Stahl, insbesondere Kupfer- oder Nickel-Stahl. Aus dem gleichen Material können der Kopf 2 mit dem Grundkörper 6 und der Ring 19 bestehen. Der Kopf 2, der Grundkörper 6 und der Ring 19 können jedoch auch aus Kunststoff gefertigt sein.

## Patentansprüche

1. Einrichtung mit mindestens einem biegsamen Führungsdraht (1) oder dergleichen zur Stabilisierung eines Bruches eines Röhrenknochens (11), wobei der Führungsdraht (1) innerhalb der Röhre des Röhrenknochens verschiebbar ist und die Einrichtung an ihrem in den Röhrenknochen (11) einzuführenden Ende einen abgerundeten Kopf (2) aufweist, und wobei die Einrichtung zusätzlich zumindest einen Verankerungsdraht (4) aufweist, **dadurch gekennzeichnet, dass** des Verankerungsdraht (4) zwischen einer Einführungsposition, bei der sein kopfseitiges Ende (5) hinter dem größten Durchmesser (3) des Kopfes (2) verborgen ist, und einer Verankerungsposition, bei der sein kopfseitiges Ende (5) über den Kopf hinausragt, verschiebbar ist, und der Kopf (2) einen Grundkörper (6) aufweist, der eine sich in Schieberichtung des Führungsdrahtes zum Kopf (2) zu erweiternde Führungsfläche aufweist, um den wenigstens einen Verankerungsdraht (4) bei Verschiebung in die Verankerungsposition zur Seite zu spreizen.

2. Einrichtung nach Anspruch 1, **dadurch gekennzeichnet, dass** die Einrichtung am abgerundeten Kopf (2) den größten Durchmesser (3) aufweist.

3. Einrichtung nach einem der Ansprüche 1 oder 2, **dadurch gekennzeichnet, dass** der Kopf (2) einen größten Durchmesser (3) zwischen 0,4 cm und 1,6 cm, vorzugsweise zwischen 0,4 cm und 1,0 aufweist.

4. Einrichtung nach Anspruch 1, **dadurch gekennzeichnet, dass** an dem vom Kopf (2) abgewandten Ende der Führungsfläche eine Führung vorgesehen ist, durch die der wenigstens eine Verankerungsdraht (4) schiebbar ist.

5. Einrichtung nach Anspruch 4, **dadurch gekennzeichnet, dass** die Führung des wenigstens einen Verankerungsdrahtes (4) durch einen den Grundkörper (6) umfassenden Ring gebildet wird, der einen kleineren Durchmesser (r) als der Kopf (2) aufweist.

6. Einrichtung nach Anspruch 5, **dadurch gekennzeichnet, dass** der wenigstens eine Verankerungsdraht (4) in einer von dem Ring nach außen geschlossenen Führungsnut (8) geführt ist.

7. Einrichtung nach Anspruch 5 oder 6, **dadurch gekennzeichnet, dass** der Ring durch eine Spiralfeder (7) gebildet wird.

8. Einrichtung nach Anspruch 7, **dadurch gekennzeichnet, dass** die Spiralfeder (7) aus dem zum Grundkörper (6) weisenden Ende des Führungsdrahtes (1) gebildet ist.

9. Einrichtung nach Anspruch 1 und 6, **dadurch gekennzeichnet, dass** die Führungsfläche durch einen bogenförmigen Abschluss (9) der Längsnut (8) am kopfseitigen Ende des Grundkörpers (6) gebildet wird.

10. Einrichtung nach Anspruch 9, **dadurch gekennzeichnet, dass** der Ring (19) mit einer Bohrung (20) zur Führung des wenigstens einen Verankerungsdrahtes (4) versehen ist.

11. Einrichtung nach Anspruch 10, **dadurch gekennzeichnet, dass** der Grundkörper (6) eine sich zum Kopf (2) hin erweiternde glatte Führungsfläche (18) aufweist.

12. Einrichtung nach einem der vorstehenden Ansprüche, **dadurch gekennzeichnet, dass** der Kopf (2) und der Grundkörper (6) einstückig ausgebildet sind.

13. Einrichtung nach einem der vorstehenden Ansprüche, **dadurch gekennzeichnet, dass** der Kopf (2) an seiner Oberfläche eine gewindeförmige Strukturierung oder ein Gewinde (17) aufweist.

## Claims

1. A device including at least one flexible guide wire (1) or the like, for stabilizing a fracture of a tubular bone (11), wherein the guide wire (1) is displaceable within the tube of the tubular bone and the device has a rounded head (2) on its end to be inserted into the tubular bone (11), and wherein the device additionally includes at least one anchoring wire (4), **characterized in that** the anchoring wire (4) is displaceable between an insertion position in which its head-side end (5) is concealed behind the largest diameter (3) of the head (2) and an anchoring position in which its head-side end (5) projects beyond the head, and that the head (2) comprises a base body (6) having a guide surface widening towards the head (2) in the direction of displacement of the guide wire so as to spread the at least one anchoring wire (4) during its displacement into the anchoring position.

2. A device according to claim 1, **characterized in that** the device has the largest diameter (3) on its rounded head (2).

3. A device according to claim 1 or 2, **characterized in that** the head (2) has its largest diameter (3) ranging between 0.4 cm and 1.6 cm, preferably between 0.4 cm and 1.0 cm.

4. A device according to claim 1, **characterized in that** the end of the guide surface facing away from the head (2) comprises a guide through which the at least one anchoring wire (4) is passable.

5. A device according to claim 4, **characterized in that** the guide of the at least one anchoring wire (4) is formed by a ring comprising the base body (6) and having a smaller diameter (r) than the head (2).

6. A device according to claim 5, **characterized in that** the at least one anchoring wire (4) is guided in a guide groove (8) which is outwardly closed by the ring.

7. A device according to claim 5 or 6, **characterized in that** the ring is comprised of a spiral spring (7).

8. A device according to claim 7, **characterized in that** the spiral spring (7) is comprised of the end of the guide wire (1) oriented towards the base body (6).

9. A device according to claims 1 and 6, **characterized in that** the guide surface is formed by a bow-shaped termination (9) of the longitudinal groove (8) on the head-side end of the base body (6).

10. A device according to claim 9, **characterized in that** the ring (19) is provided with a bore (20) for guiding the at least one anchoring wire (4).

11. A device according to claim 10, **characterized in that** the base body (6) comprises a smooth guide surface (18) widening towards the head (2).

12. A device according to any one of the preceding claims, **characterized in that** the head (2) and the base body (6) are made in one piece.

13. A device according to any one of the preceding claims, **characterized in that** the head (2) comprises a thread-shaped structure or thread (17) on its surface.

## Revendications

1. Dispositif avec au moins un fil de guidage flexible (1) ou similaire pour stabiliser une fracture d'un os long (11), le fil de guidage (1) pouvant être déplacé à l'intérieur du canal de l'os long et le dispositif présentant à son extrémité à introduire dans l'os long (11) une tête (2) arrondie, et le dispositif présentant en outre au moins un fil d'ancrage (4), **caractérisé en ce que** le fil d'ancrage (4) peut être déplacé entre une position d'introduction dans laquelle son extrémité (5) du côté de la tête est cachée derrière le plus grand diamètre (3) de la tête (2) et une position d'ancrage dans laquelle son extrémité (5) du côté de la tête fait saillie au-delà de la tête, et la tête (2) présente un corps de base (6) qui présente une surface de guidage s'élargissant dans le sens de déplacement du fil de guidage en direction de la tête (2) pour écarter sur le côté ledit au moins un fil d'ancrage (4) lorsqu'il est déplacé dans la position d'ancrage.

2. Dispositif selon la revendication 1, **caractérisé en ce que** le dispositif présente le plus grand diamètre (3) sur la tête (2) arrondie.

3. Dispositif selon la revendication 1 ou 2, **caractérisé en ce que** la tête (2) présente un plus grand diamètre (3) compris entre 0,4 cm et 1,6 cm, de préférence entre 0,4 cm et 1,0 cm.

4. Dispositif selon la revendication 1, **caractérisé en ce qu'**il est prévu sur l'extrémité de la surface de guidage opposée à la tête (2) un guidage grâce à laquelle ledit au moins un fil d'ancrage (4) peut être déplacé.

5. Dispositif selon la revendication 4, **caractérisé en ce que** le guidage dudit au moins un fil d'ancrage (4) est formé par une bague entourant le corps de base (6) qui présente un plus petit diamètre (r) que la tête (2).

6. Dispositif selon la revendication 5, **caractérisé en ce que** ledit au moins un fil d'ancrage (4) est guidé dans une rainure de guidage (8) fermée vers l'extérieur par la bague.

7. Dispositif selon la revendication 5 ou 6, **caractérisé en ce que** la bague est formée par un ressort en spirale (7).

8. Dispositif selon la revendication 7, **caractérisé en ce que** le ressort en spirale (7) est formé à partir d'une extrémité du fil de guidage (1) orientée vers le corps de base (6).

9. Dispositif selon la revendication 1 et 6, **caractérisé en ce que** la surface de guidage est formée par une terminaison (9) en forme d'arc de la rainure longitudinale (8) sur l'extrémité du corps de base (6) du côté de la tête.

10. Dispositif selon la revendication 9, **caractérisé en ce que** la bague (19) est pourvue d'un orifice (20) pour le guidage dudit au moins un fil d'ancrage (4).

11. Dispositif selon la revendication 10, **caractérisé en ce que** le corps de base (6) présente une surface de guidage (18) lisse s'élargissant en direction de la tête (2).

12. Dispositif selon l'une quelconque des revendications précédentes, **caractérisé en ce que** la tête (2) et le corps de base (6) sont formés d'un seul tenant.

13. Dispositif selon l'une quelconque des revendications précédentes, **caractérisé en ce que** la tête (2) présente une structure en forme de filetage ou un filetage (17) sur sa surface.
